# EUROPÄISCHE PATENTSCHRIFT

(11) **EP 3 073 936 B1**
(45) Veröffentlichungstag und Bekanntmachung des Hinweises auf die Patenterteilung: **26.07.2023**
(21) Anmeldenummer: 14820761.6
(22) Anmeldetag: 28.11.2014
(51) Int. Cl.: A61B 17/12, A61B 17/00

(54) **MEDIZINISCHES IMPLANTAT**
MEDICAL IMPLANT
IMPLANT MÉDICAL

(30) Priorität: 28.11.2013 DE 102013019890
(43) Veröffentlichungstag der Anmeldung: 05.10.2016
(73) Patentinhaber: Bentley InnoMed GmbH, 72379 Hechingen (DE)
(72) Erfinder: OBRADOVIC, Milisav, 79539 Lörrach (DE)
(74) Vertreter: Hoefer & Partner Patentanwälte mbB
(86) Internationale Anmeldenummer: PCT/EP2014/075942
(87) Internationale Veröffentlichungsnummer: WO 2015/079023

(56) Entgegenhaltungen:
- EP-A1- 2 338 420
- EP-A1- 3 120 786
- WO-A1-2012/166804
- CN-U- 202 143 640
- US-A1- 2003 220 667
- US-A1- 2004 122 467
- US-A1- 2011 054 515

## Beschreibung

Die Erfindung betrifft medizinisches Implantat zum Verschließen der auricula sinistra eines Patienten auf endovaskulärem Weg, das eine Korbstruktur aus einer Vielzahl von Stegen aufweist, die proximal über Verbindungsstege mit einem Haltering verbunden ist und distal von einem Kranz zusammenlaufender Stege begrenzt ist, wobei das Implantat aus einem selbstexpandierenden Material besteht, in einem kontrahierten Zustand die Form eines geschlitzten Rohrs hat und nach der Expansion eine Korbstruktur mit einem gegenüber dem Haltering erweiterten Durchmesser annimmt.

Die auriculae atrii oder Vorhofohren sind Ausstülpungen der Vorhöfe des Herzens bei Säugetieren. Das linke Vorhofohr, medizinisch als auricula sinistra bezeichnet, liegt neben dem Strang der Lungenarterie und ist, insbesondere bei Patienten mit Vorhofflimmern, ein häufiger Entstehungsort für Blutgerinnsel, die zu einem Schlaganfall führen können. Die Verhinderung von Thromben in der auricula sinistra stellt daher eine wirksame Schlaganfallprophylaxe bei gefährdeten Patienten dar.

Für diese Schlaganfallprophylaxe wurden Implantate entwickelt, die in den Ausstülpungen eingesetzt werden und den Zugang zumeist über ein Geflecht oder eine Folie verschließen. In der angelsächsischen Literatur werden diese Implantate als LAA-Okkluder bezeichnet (LAA: left atrial appendage). Diese Implantate werden in die Ausstülpungen eingesetzt und dort über Verspannungselemente verankert; sie schließen mit ihrem proximalen Ende den Zugang ab. Die Einbringung erfolgt zumeist über endovaskuläre Techniken d.h. mit einem Katheter, durch den das Implantat in volumenreduzierter Form an den Einsatzort verbracht, dort aus dem Katheter ausgebracht und expandiert wird. Für die Expansion werden in der Regel selbstexpandierende Materialien verwandt, beispielsweise Formgedächtnislegierungen.

Die richtige und zuverlässige Verankerung der Implantate ist häufig ein Problem. Größe und Form der auricula sinistra kann von Patient zu Patient unterschiedlich sein und insbesondere hinsichtlich der Zugangsöffnung enger und weiter ausfallen. Implantate, die durch die Expansion mit den Wandungen der auricula verspannt werden, können deshalb bei nicht optimalem Sitz verrutschen und hierdurch ihren Zweck nicht optimal erfüllen. In solchen Fällen kann es immer noch zur Abschwemmung von Thromben kommen, insbesondere bei körperlicher Belastung des Patienten.

US 2003/220667 A1 offenbart ein Implantat zum Verschließen der auricula sinistra, welches einen selbst-expandierenden Rahmen aufweist.

US 2004/122467 A1 offenbart ebenfalls ein Implantat zum Verschließen der auricula sinistra, welches einen selbst-expandierenden Rahmen aufweist, wobei der Rahmen am distalen Ende Ankerelemente aufweist.

US 2011/054515 A1 und CN 202 143 640 U offenbaren jeweils ein Implantat zum Verschließen der auricula sinistra, welches einen korbförmigen Rahmen aufweist, wobei der Rahmen Ankerelemente aufweist.

Angesichts dessen ist es Aufgabe der Erfindung, ein Implantat für die auricula sinistra bereitzustellen, das einen zuverlässigen Sitz und eine optimale Abschirmung gegen den Blutkreislauf gewährleistet.

Diese Aufgabe wird mit einem Implant der eingangs genannten Art gelöst, dass ein oder mehrere Ankerelemente aufweist, welche proximal direkt mit dem Haltering verbunden sind, wobei
a) das oder die Ankerelemente distal eine Spitze mit Widerhaken aufweisen, mit Spitze und Widerhaken über die Korbstruktur hinausragen und dazu bestimmt sind, mit Spitze und Widerhaken im Muskelgewebe der auricula sinistra verankert zu werden, oder
b) das oder die Ankerelemente lateral über die Korbstruktur hinausragen, nach proximal gekrümmt verlaufen und dazu bestimmt sind, sich gegen das Muskelgewebe der auricula sinistra seitlich abzustützen, oder
c) eine Kombination der Alternativen (a) und (b).

Alle Alternativen (a) bis (c) sind geeignet, einen zuverlässigen Sitz des Implantats in der der auricula sinistra zu gewährleisten, dadurch, dass ein Fixierung des Implantats in der der auricula sinistra gegeben ist. Das erfindungsgemäße Implantat deckt lediglich Alternative (b) ab.

Das erfindungsgemäße Implantat weist einen Haltering auf, der über eine Mehrzahl von Stegen mit einer distal zum Haltering angeordneten Korbstruktur verbunden ist. Die Korbstruktur ist im expandierten Zustand des Implantats gegenüber dem Haltering stark erweitert. Sie wird von einer Vielzahl von Stegen gebildet, die zweckmäßigerweise eine Maschen- oder Netzstruktur ausbilden. Während die Korbstruktur proximal in dem Haltering ausläuft, ist sie distal offen und wird von einem Kranz zickzackförmig angeordneter und zusammenlaufender Stege begrenzt. Insbesondere besteht die Korbstruktur aus einem Netzwerk von sich verzweigenden und wieder zusammenlaufenden Stegen.

Zur Verankerung im Muskelgewebe der auricula sinistra gemäß Alternative (a) weist das Implantat ein oder mehrere Ankerelemente auf, die über die Korbstruktur hinausragen. Jedes Ankerelement endet in einer Spitze mit einem Widerhaken, die dazu bestimmt sind, im Muskelgewebe einzuhaken.

Zur Verankerung in der auricula sinistra gemäß Alternative (b) weist das erfindungsgemäße Implantat mehrere Ankerelemente auf, die lateral aus der Korbstruktur hinausragen, nach proximal gekrümmt verlaufen und dazu bestimmt sind, sich gegen das Muskelgewebe der auricula sinistra seitlich abzustützen. In diesem Fall bilden die Ankerelemente einen zweiten nach proximal offenen Korb, wobei die Enden der Ankerelemente den Rand des Korbes definiert und sich am Muskelgewebe seitlich vom Eingang der auricula sinistra abstützen. Auch in diesem Fall können die Enden der Ankerelemente, gegebenenfalls auch Widerhaken aufweisen, jedoch ist es in der Regel ausreichend, dass die Spitzen abgerundet sind. Auch im letzteren Fall folgt eine sichere Abstützung durch die Verspreizung in der auricula sinistra.

Die Ankerelemente gehen direkt vom Haltering aus und ragen über die Korbstruktur hinaus, sodass sie mit dem Muskelgewebe der auricula sinistra in Kontakt gebracht werden können. Gemäß einer Ausführungsform der Alternative (a) befinden sich das oder die Ankerelemente an den distalen Spitzen des Kranzes der Korbstruktur. Dabei können die Widerhaken nach außen oder nach innen weisen; vorzugsweise sind die Widerhaken an der Außenseite angeordnet. Sind mehrere Ankerelemente vorhanden, sind diese vorzugsweise gleichmäßig über den Kranz verteilt, wobei nicht jede Spitze des Kranzes ein Ankerelement aufweisen muss.

Bevorzugt ist aber eine Variante gemäß Alternative (a), bei der das Ankerelement oder jedes Ankerelement von dem Haltering des Implantats ausgeht und durch die Korbstruktur hindurch verläuft und distal über diese hinausragt. Das oder die Ankerelemente befinden sich damit in etwa der Mitte der Korbstruktur. Auch hier ist eine regelmäßige Verteilung der Ankerelemente bevorzugt, sofern mehrere Ankerelemente vorgesehen sind.

Das erfindungsgemäße medizinische Implantat wird über einen üblichen Katheter an seinen Einsatzort transportiert und dort aus dem Katheter freigesetzt. Im Katheter liegt es in einer volumenreduzierten, kontrahierten und gestreckten Form vor und hat im Wesentlichen die Form eines mehrfach geschlitzten Rohres. Die Form entspricht dem Rohr, aus dem das Implantat durch Laserschneiden erzeugt wurde.

Nach der Freisetzung aus dem Katheter nimmt das Implantat die expandierte Form an, die ihm durch ein Temperverfahren aufgeprägt worden ist, d. h. die Korbform mit den darüber hinaus ragenden Ankerelementen.

Zur Platzierung ist das im Katheter geführte Implantat über den Haltering mittels eines Kupplungsmechanismus mit einem Führungselement verbunden, vorzugsweise einem Führungskatheter oder -draht. Bei der Implantierung wird das Implantat dann mittels Führungselement in die auricula sinistera hineingeschoben und zur Expansion gebracht. Nach der Expansion sind die im Zentrum der Korbstruktur angeordneten Ankerelemente mit ihren Spitzen und Widerhaken außerhalb des Korbes. Mittels Führungskatheter und/oder Führungsdraht kann der erforderliche Druck ausgeübt werden, um die Spitzen mit den Widerhaken in dem Muskelgewebe an der hinteren Wand der auricula zu verankern. Die Spitzen mit den Widerhaken wachsen problemlos ein und halten das Implantat in der einmal gewählten Stellung zuverlässig fest. Nach der Platzierung wird das Führungselement auf übliche Art und Weise vom Implantat gelöst und zusammen mit dem Katheter zurückgezogen. Derartige Kupplungsmechanismen sind bekannt und vielfach beschrieben.

Die Korbstruktur des erfindungsgemäßen medizinischen Implantats ist in der Regel über 6 bis 12 Verbindungsstege mit dem Haltering verbunden. Bewährt hat sich eine Anzahl von 8 oder 10 Verbindungsstegen, die sich anschließend zur Korbstruktur verzweigen und distal zum abschließenden Kranz erneut vereinigen.

Das erfindungsgemäße Implantat kann bei hinreichender Dichte der Stege seinen Zweck als Thrombenfilter auch ohne eine Abdeckung oder Bespannung erfüllen. Zweckmäßigerweise ist das medizinische Implantat im proximalen Bereich aber mit einer Abdeckung versehen, beispielsweise eine Polyurethan-, Polyester oder Teflonfolie. Um eine solche Abdeckung an der Korbstruktur festzulegen, ist es zweckmäßig, die vom Haltering ausgehenden Verbindungsstege mit Perforationen zu versehen, die zum Vernähen der Abdeckung verwandt werden können. Die Abdeckung kann aber auch durch Verkleben festgelegt werden oder durch (wiederholtes) Tauchen des Implantates in eine Kunststofflösung oder -dispersion erzeugt werden.

Die Implantate weisen vorzugsweise im Inneren des Korbs ein oder mehrere Ankerelemente auf, die, ausgehend vom Haltering (a) speerförmig aus der Öffnung der Korbstruktur herausragen und eine Spitze mit wenigstens einem Widerhaken aufweisen. Spitze und Widerhaken befinden sich außerhalb der Korbstruktur und sind geeignet, bei geeigneter Druckausübung bei der Platzierung mittels Führungsdraht in das Muskelgewebe der auricula sinistra einzudringen und sich dort zu verhaken. Gemäß Alternative (b) ragen die Ankerelemente lateral über die Korbstruktur hinaus und sind nach proximal gekrümmt, so dass sie das Implantat gegen die Seitenwand im Eingangsbereich der auricula sinistra abstützen. Eine Kombination der beiden Varianten (a) und (b) ist ebenfalls möglich. Auf diese Art und Weise wird das Implantat zuverlässig so fixiert, dass sich der proximale Teil des Korbs mit dem Haltering im Eingangsbereich befindet. Der Korb mit oder ohne Abdeckung schirmt damit die auricula sinistra ab und verhindert das Ausschwemmen von Thromben.

Entsprechendes gilt bei Anordnung der Ankerelemente am Korbkranz; in diesem Fall bilden die Ankerelemente das distale Ende des Korbes.

Im Prinzip ist ein Ankerelement gemäß Alternative (a) ausreichend, vorzugsweise weist das Implantat aber zwei oder mehr Ankerelemente auf, die gleichmäßig über den Umfang des Halterings verteilt sind. Besonders bevorzugt ist eine Anordnung mit zwei Ankerelementen, die einander am Haltering gegenüberstehen. Die Ankerelemente im Zentrum des Korbes verlaufen im Wesentlichen parallel zueinander in etwa der Mitte der Korbstruktur.

Gemäß Alternative (b) ist eine Vielzahl von Ankerelementen zweckmäßig, wobei sie die Zahl an der Zahl der Verbindungsstege zwischen Haltering und Korb orientiert. Auch hier sind die Ankerelemente gleichmäßig über den Umfang des Halterings verteilt. Zweckmäßigerweise sind sie zwischen jeweils zwei Verbindungsstegen angeordnet.

Wie schon dargestellt weist die Korbstruktur vorzugsweise ein Netzwerk sich verzweigender und zusammenlaufender Stege auf, die rautenförmige Strukturen ausbilden. Durch zusammenlaufende Stege im distalen Bereich entsteht ein Kranz zusammenlaufender Stege mit zickzackförmigem Verlauf, dessen Spitzen vorzugsweise abgerundet sind. Ein mäandrierender, gerundeter Verlauf ist ebenfalls möglich. Bei Anordnung der Ankerelemente am Kranz ist ein zickzackförmiger Verlauf mit auf die Spitzen des Kranzes aufgesetzten Ankerelementen bevorzugt.

Am proximalen Ende der Korbstruktur ist der zentrale Haltering vorzugsweise in einer zentralen Eintiefung der Korbstruktur angeordnet. Dies bedeutet, dass die Verbindungsstege zwischen Haltering und Korbstruktur einen S-förmigen verlaufen nehmen, d. h. ausgehend von der distalen Seite des Halterings sich zunächst nach proximal wenden, bevor sie sich erneut nach distal orientieren und in die Korbstruktur übergehen.

Gemäß einer weiteren Ausführungsform der Erfindung sind die Ankerelemente distal zur Korbstruktur angeordnet und erstrecken sich lateral über die Korbstruktur hinaus. In diesem Fall gehen die Ankerelemente von einem Halterohr aus, das seinerseits mit dem Haltering der Korbstruktur verbunden ist. Das Halterohr hat zweckmäßigerweise einen etwas geringeren Durchmesser als der Haltering und ist in diesen eingepasst und mit dem Haltering verbunden, etwa verschweißt.

Die Ankerelemente sind in dieser Ausführungsform vorzugsweise paarweise miteinander an den Spitzen verbunden, so dass sie eine Art Schlaufe bilden. Dabei können die Spitzen auf den Korb zurückgebogen sein, so dass sich die Ankerelemente mit ihren Außenflächen an der Peripherie gegen die auricula sinistra verspannen.

Die erfindungsgemäßen Implantate bestehen aus einen flexiblen, selbst expandierenden Material. Dieses Material kann ein Metall oder ein Kunststoff sein, ist zweckmäßigerweise aber eine Metalllegierung mit Formgedächtniseigenschaften. Besonders bevorzugt sind Nickel-Titan-Legierungen, beispielsweise Nitinol. Die Herstellung von Implantaten aus diesen Materialien und die Umformung durch Tempern sind vielfach beschrieben. Derartige Formgedächtnismetalle sind in der Lage, unter äußerem Zwang die ursprüngliche herstellungsbedingte Form einzunehmen und bei Wegfall dieses Zwangs eine später aufgeprägte Form, die durch Tempern fixiert wird, erneut einzunehmen. Dies erlaubt den Transport solcher Implantate in einem Katheter mit geringem Durchmesser und die anschließende Expansion nach Freisetzung aus dem Katheter.

Entsprechend werden auch die erfindungsgemäßen Implantate durch Laserschneiden aus einem Rohr gefertigt und durch anschließendes thermisches Umformen in die expandierte Form gebracht.

Die erfindungsgemäßen Implantate, wenn aus einer Form Gedächtnislegierung gefertigt, können auch aus zwei Teilen bestehen, die aus separaten Rohren geschnitten werden. So kann aus einem Rohr der Korb gefertigt werden und aus einem zweiten Rohr die Ankerelemente, wobei in jedem Fall ein Haltering vorhanden ist. Die beiden Teile werden zu einem Implantat verbunden, insbesondere verschweißt.

Die Erfindung wird durch die beiliegenden Abbildungen näher erläutert. Es zeigen:
- Fig. 1:: Ein Schnittmuster für ein Implantat gemäß Alternative (a) in flächiger Darstellung;
- Fig. 2:: eine seitliche Darstellung eines aus einem Rohr gemäß Fig. 1 geformten Implantats;
- Fig. 3:: eine Darstellung des Implantats gemäß Fig. 2 von der proximalen Seite;
- Fig. 4:: eine seitliche Darstellung einer weiteren Ausführungsform eines Implantats gemäß Alternative (a);
- Fig. 5:: eine fotografische Darstellung einer Ausführungsform eines erfindungs-gemäßen Implantats gemäß Alternative (b);
- Fig. 6:: eine weitere Fotografie des Implantats gemäß Fig. 5, angekoppelt an einen Führungsdraht;
- Fig. 7:: eine weitere Ausführungsform eines erfindungsgemäßen Implantats mit distal zur Korbstruktur angeordneten Ankerelementen; und
- Fig. 8:: eine seitliche Darstellung des Implantats von Fig. 7.

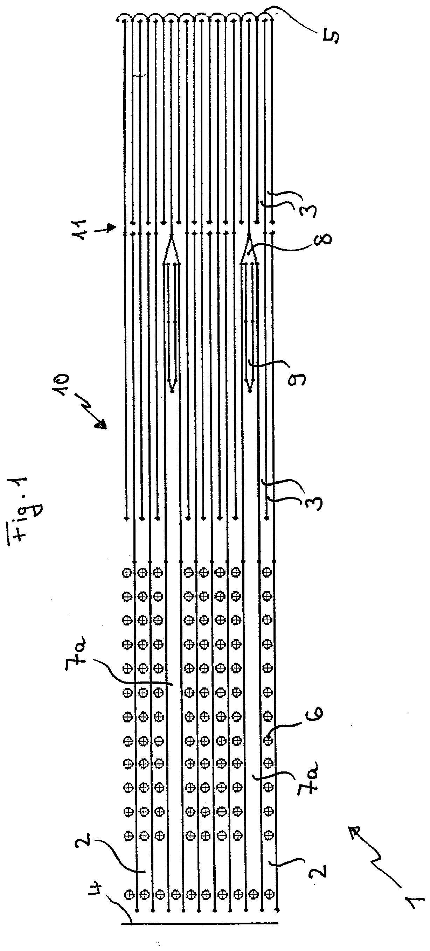

Fig. 1 zeigt ein Schnittmuster für ein Implantat gemäß Alternative (a) in flächiger Darstellung, d. h. in aufgeschnittener und ausgebreiteter Form, vor der thermischen Umformung. Die Darstellung zeigt somit das ursprünglich bearbeitete Rohr in flächiger Darstellung.

Von einem Haltering 4 geht eine Mehrzahl von Verbindungsstegen 2 aus, die jeweils mehrere Perforationen 6 zeigen, die dazu dienen, eine Bespannung, beispielsweise mit einer Teflonfolie, daran zu vernähen. Die Verbindungsstege 2 gehen jeweils in zwei Korbstege 3 über, die in einer Kreuzungsebene 11 miteinander vernetzt sind. Im distalen Bereich der Korbstruktur 10 laufen jeweils zwei Korbstege 3 zu einer abgerundeten Spitze 5 zusammen, wobei die Gesamtzahl der Spitzen 5 die kranzförmige distale Begrenzung des Korbs 10 ausbilden.

Ausgehend vom Haltering 4 sind zwei Ankerelemente 7a ausgeschnitten, die in einer Spitze 8 enden. Durch Einschnitte wird ein Hakenelement 9 definiert, das in umgeformtem Zustand den Widerhaken 9 der Spitze 8 ausbildet.

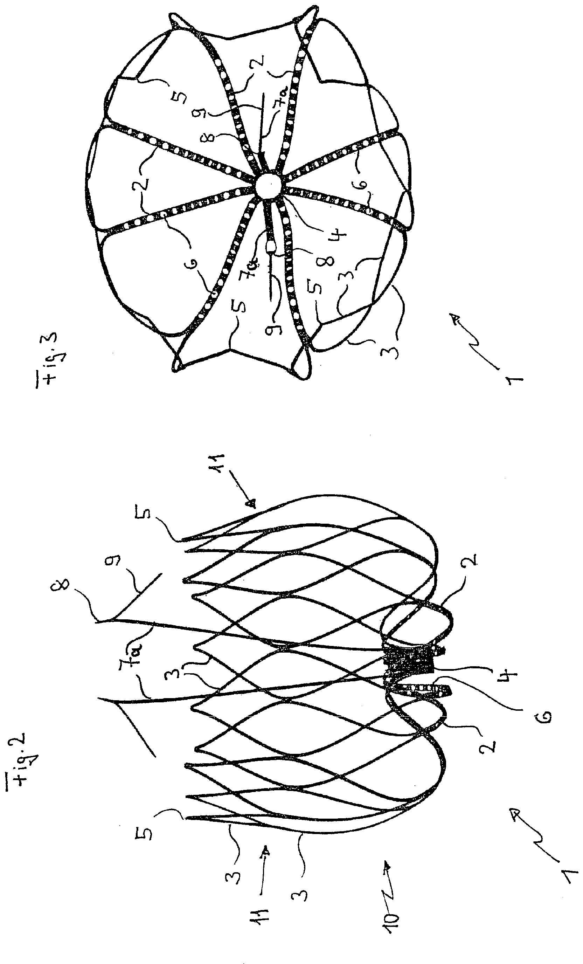

Fig. 2 zeigt ein Implantat gemäß Alternative (a) in seitlicher photographischer Wiedergabe mit dem Haltering 4 und der Korbstruktur 10. Vom Haltering geht eine Mehrzahl von Verbindungsstegen 2 aus, die sich zur Korbstruktur 10 mit ihren Korbstegen 3 verzweigen. Die Korbstege 3, die von den Verbindungsstegen 2 ausgehen, laufen in einer Ebene 11 zusammen, um sich erneut zu verzweigen und wieder zusammenzulaufen. An den Verbindungsstellen im distalen Bereich des Korbes werden Spitzen 5 ausgebildet, die vorzugsweise abgerundet gestaltet werden.

Vom Haltering 4 gehen zwei speerförmig ausgebildete Ankerelemente 7a aus, die im Bereich ihrer Spitze 8 einen Widerhaken 9 ausbilden. Diese Ankerelemente ragen über die Korbstruktur 10 hinaus und dienen dazu, das Implantatmuskelgewebe der auricula sinistra gegenüber dem Eingang zu verankern. Die Korbstruktur 10 kommt dabei an den Wänden zu liegen, das proximale Ende mit dem Haltering 4 ist im Eingangsbereich angeordnet.

Die Verbindungsstege 2, die den Haltering 4 mit der Korbstruktur 10 und seinen Stegelementen verbinden, haben einen mehrfach gekrümmten Verlauf, dergestalt, dass sie zunächst nach proximal verlaufen und anschließend halbkreisförmig zum Korb 10 nach distal umgebogen sind. Die Stege 2 weisen Perforationen 6 zur Festlegung einer Abdeckung auf, wie in Fig. 1 gezeigt.

Erfindungsgemäße Implantate sind über den Haltering 4 mit einem üblichen Kupplungsmechanismus mit einem Führungskatheter verbunden, der durch einen Katheter zum behandelnden Arzt führt. Diese Techniken sind allgemein bekannt und vielfach beschrieben. Es versteht sich auch, dass der Haltering 4 zur Verhinderung der Ausschwemmung von Thromben auf eine geeignete Weise verschlossen sein kann, beispielsweise durch eingepasste Elemente, aus dem Haltering ausgeklinkte Streben oder den in den Haltering eingepassten Kupplungsmechanismus, etwa in Form einer Platte oder Kugel, die mit einem zangenförmigen Halteelement des Führungskatheters oder -drahts zusammenwirken.

Fig. 3 zeigt das Implantat gemäß Fig. 2 von der proximalen Seite in photographischer Wiedergabe. Der Haltering 4 ist über acht Verbindungsstege 2 mit dem eigentlichen Korb verbunden, der durch die Korbstege 3 gebildet wird. Jeder Verbindungssteg 2 geht in zwei Stegen 3 über, die den Korb in Form eines Netzwerks ausbilden. Die Peripherie der Fig. 3 wird durch die Maschenstruktur des Korbs gebildet, die in dem von den Spitzen 5 gebildeten Kranz distal endet.

Die Verbindungsstege 2 weisen eine Vielzahl von Perforationen 6 aus, die dazu dienen, eine das Implantat bedeckende Abdeckung festzulegen, im gezeigten Fall durch Festnähen. Eine solche Abdeckung kann beispielsweise aus einer Teflonfolie bestehen. Es ist aber ohne Weiteres möglich, die Abdeckung auch zu verkleben, zu verspannen oder durch Tauchen oder Elektrospinnen mit der Implantatstruktur zu verbinden.

Vom Haltering 4 gehen zentral die Halteelemente 7a aus, die in einer Spitze 8 enden. Die Widerhaken 9 gehen von der Spitze 8 aus und weisen im dargestellten Fall zur Peripherie des Korbs. Zu erkennen ist in dem Halteelement links der Ausschnitt, aus dem der Widerhaken 9 ausgeklinkt wurde.

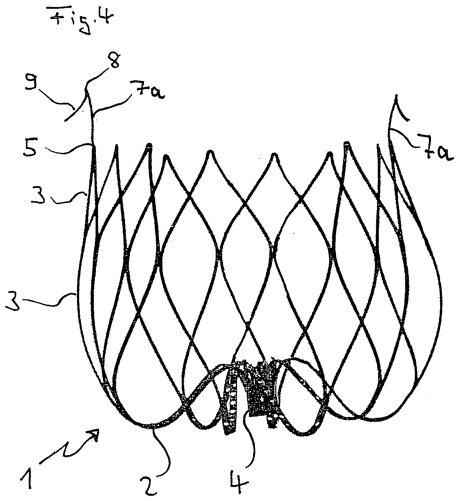

Fig. 4 zeigt eine weitere Variante eines Implantats gemäß Alternative (a), bei dem die Halteelemente 7 mit Spitze 8 und Widerhaken 9 von Spitzen 5 der Kranzsstruktur des Implantats ausgehen. Nicht jede Spitze ist mit einem Halteelement verbunden; vorzugsweise sind zwei oder vier Halteelemente vorhanden, die in regelmäßigen Abständen angeordnet sind.

Der Begriff "proximal" im Sinne der Beschreibung bezeichnet die zum behandelnden Arzt und Katheter weisende Seite des Implantats, der Begriff "distal" die vom behandelnden Arzt und Katheter wegweisende und zur hinteren Wand der auricula sinistra weisende Seite des Implantats.

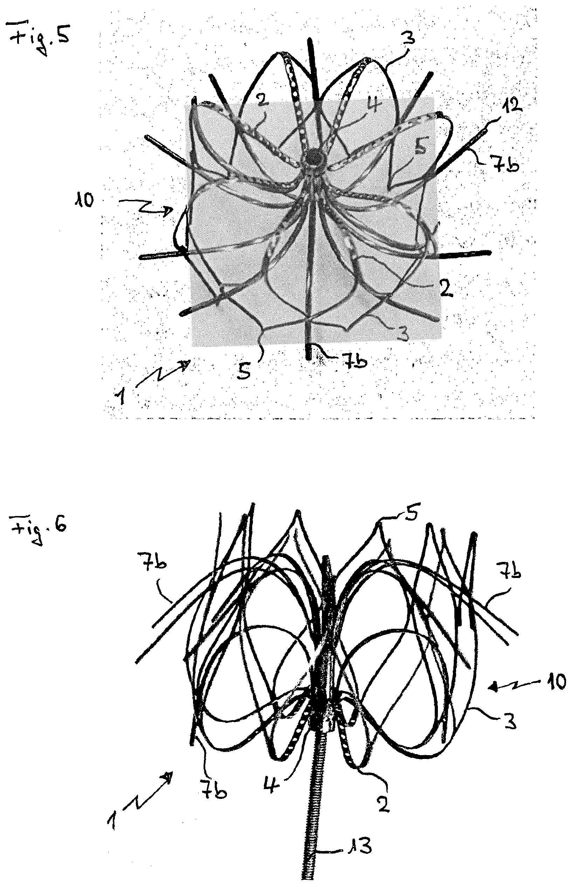

Fig. 5 zeigt ein erfindungsgemäßes Implantat gemäß Alternative (b). Das Implantat 1 weist aus dem Haltering 4 hervorgehende Verbindungsstege 2 auf, die sich in die Stege 3, die den eigentlichen Korb 10 bilden, aufgabeln. Die Stege 3 sind miteinander vernetzt und enden in Spitzen 5, die athromatisch abgerundet sind.

Die Variante (b) zeichnet sich durch Ankerelemente 7b aus, die zwischen den Verbindungsstegen 2 vom Haltering 4 ausgehen und sich lateral nach außen strecken und dabei den Bereich des Korbs 10 verlassen. Die Ankerelemente 7b haben einen gekrümmten Verlauf d. h. sie sind nach proximal gekrümmt. Auf diese Art und Weise bilden sie eine zum Korb 10 gegenläufigen Korb aus. Sie bilden ein Kranz von Armen, die in der auricula sinistra gegen die Wand stoßen und das Implantat dabei fixieren.

Fig. 6 zeigt eine weitere Fotografie des Implantats von Fig. 5, in diesem Fall verbunden mit einem Führungsdraht 13, der nach der Implantation durch Verdrehen gelöst werden kann. Deutlich zu erkennen ist die von den Verbindungsstegen 2 und den Stegen 3 gebildete Korbstruktur, die in den Spitzen 5 distal zum Führungsdraht 13 endet. Die Ankerelemente 7b gehen, wie die Verbindungsstege 2, vom Haltering 4 aus, sind zwischen benachbarten Verbindungsstegen 2 angeordnet und haben einen gekrümmten Verlauf dergestalt, dass sie sich aus dem Korb 10 nach außen erstrecken. Die Krümmung der Ankerelemente 7b führt dazu, dass sie in ihrem Endbereich einen nach außen und rückwärts gerichteten (proximal) Verlauf annehmen. Hierauf beruht die Sperrwirkung, die das Implantat am Implantationsort in der auricula sinistra festhält.

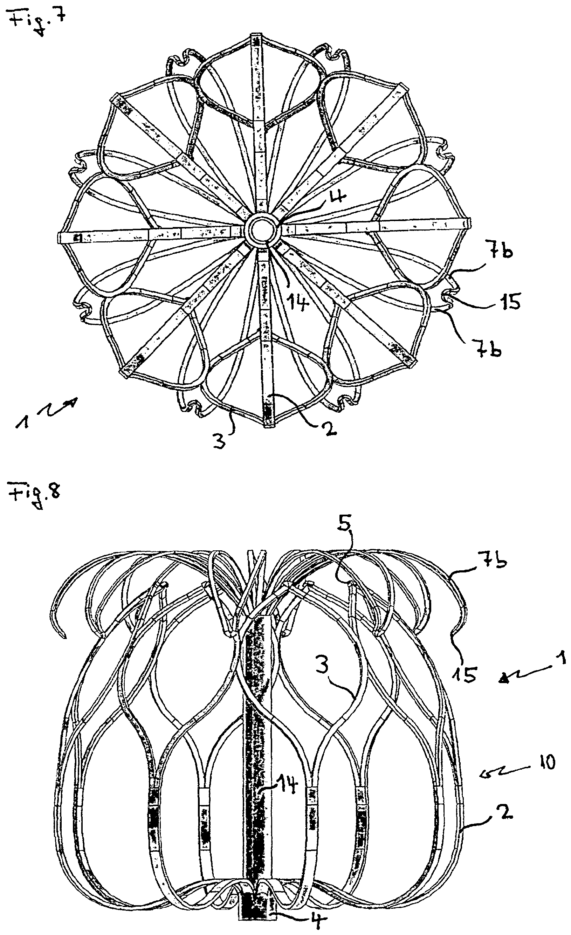

Fig. 7 zeigt eine weitere Variante des erfindungsgemäßen Implantats 1 von proximal. Ausgehend von dem Haltering 4 bilden die Stege 2 und 3 den Korb 10 (nicht gesondert bezeichnet). Die Ankerelemente 7b gehen von einem Halterohr 14 aus, das an seinem proximalen Ende in den Haltering 4 eingepasst und mit diesem verbunden ist. Am distalen Ende des Halterohrs 14 beginnen die Ankerelemente 7b, von denen jeweils zwei benachbarte zu einer Schlaufe zusammengefasst sind, die in einer gerundeten Spitze 15 endet. Die Spitze der Ankerelemente 7b ist einwärts gebogen, d. h. auf den Korb zurückgebogen. Das Ankerelement 7b ist dazu bestimmt, sich peripher mit der Außenseite in der auricula sinistra zu verankern bzw. zu verspreizen. Die einwärts gekrümmte Spitze 15 der von zwei Elementen 7b gebildeten Schlaufe verhindert zum einen eine Verletzung des Gewebes und bewirkt zum anderen eine Spannung der Ankerelemente 7b, die der Verankerung des Implantats nutzt.

Fig. 8 ist eine Darstellung des Implantats an Fig. 7 von der Seite her. Zu erkennen ist das Halterohr 14, das mit seinem proximalen Ende in den Haltering 4 eingepasst ist. Der Haltering 4 ist der Ausgangspunkt der Korbstruktur 10, die aus den Stegen 2 und 3 gebildet wird, wobei die Stege 3 in Spitzen 5 zusammengeführt sind.

Die Ankerelemente 7b gehen distal vom Halterohr 14 aus und erstrecken sich lateral über die Korbstruktur hinaus mit einer Krümmung zur proximalen Seite des Implantats 1, wobei die Enden der Ankerelemente 7b auf den Korb zurückgekrümmt sind. Jeweils zwei benachbarte Ankerelemente 7b bilden eine Schlaufe, die in einer in Richtung auf den Korb zeigenden Spitze 15 auslaufen. Die Ankerelemente 7b verspannen sich peripher gegen die Wandung der auricula sinistra und sorgen so für den Erhalt des Implantats am Einsatzort.

## Patentansprüche

1. Medizinisches Implantat (1) zum Verschließen der auricula sinistra eines Patienten auf endovaskulärem Weg, das eine Korbstruktur (10) aus einer Vielzahl von Stegen (3) aufweist, die proximal über Verbindungsstege (2) mit einem Haltering (4) verbunden ist und die distal offen ist und von einem Kranz zusammenlaufender Stege (3) begrenzt ist, wobei das Implantat (1) aus einem selbstexpandierenden Material besteht, in einem kontrahierten Zustand die Form eines geschlitzten Rohrs hat und nach der Expansion die Korbstruktur (10) mit einem gegenüber dem Haltering (4) erweiterten Durchmesser annimmt, wobei nach der Expansion innerhalb der Korbstruktur (10) oder distal zur Korbstruktur (10) mehrere Ankerelemente (7) angeordnet sind, die proximal direkt mit dem Haltering (4) verbunden sind, und die Ankerelemente (7b) lateral über die Korbstruktur (10) hinausragen, nach proximal gekrümmt verlaufen und dazu bestimmt sind, sich gegen das Muskelgewebe der auricula sinistra seitlich abzustützen.

2. Implantat nach Anspruch 1, wobei die Ankerelemente (7b) gleichmäßig über den Umfang des Halterings (4) angeordnet sind.

3. Implantat nach einem der vorstehenden Ansprüche, wobei die Ankerelemente (7b) innerhalb der Korbstruktur angeordnet und direkt mit dem Haltering (4) verbunden sind.

4. Implantat nach einem der vorstehenden Ansprüche, wobei die vom Haltering (4) ausgehenden Verbindungsstege (2) zur Korbstruktur (10) Perforationen (6) aufweisen.

5. Implantat nach einem der vorstehenden Ansprüchen, wobei das Implantat weiterhin eine Abdeckung im proximalen Bereich aufweist.

6. Implantat nach Anspruch 5, wobei die Abdeckung aus einer Teflonfolie besteht.

7. Implantat nach Anspruch 5 oder 6, wobei die Abdeckung über die Perforationen mit der Korbstruktur (10) vernäht ist.

8. Implantat nach einem der vorstehenden Ansprüche, wobei die Korbstruktur (10) von einem Netzwerk sich verzweigender und zusammenlaufender Stege (3) gebildet wird.

9. Implantat nach einem der vorstehenden Ansprüche, wobei der distale Kranz der Korbstruktur (10) abgerundete Spitzen (5) aufweist.

10. Implantat nach einem der vorstehenden Ansprüche, wobei das Implantat weiterhin eine Kupplung für einen Führungskatheter am Haltering (4) aufweist.

11. Implantat nach einem der vorstehenden Ansprüche, wobei die Korbstruktur (10) im proximalen Bereich eine zentrale Eintiefung aufweist, in der der Haltering (4) angeordnet ist.

12. Implantat nach einem der vorstehenden Ansprüche, wobei die Ankerelemente (7b) jeweils zwischen zwei Verbindungsstegen (2) am Haltering (4) angeordnet sind.

13. Implantat nach einem der vorstehenden Ansprüche, wobei die Ankerelemente (7b) abgerundete Enden (12) aufweisen.

14. Implantat nach Anspruch 1, wobei die Ankerelemente (7b) distal zur Korbstruktur (10) angeordnet sind, lateral über die Korbstruktur (10) hinausragen und über ein Halterohr (14) mit dem Haltering (4) verbunden sind.

15. Implantat nach Anspruch 14, wobei benachbarte Ankerelemente (7b) paarweise miteinander verbunden sind, wobei die Spitzen der Ankerelemente (7b) in Richtung auf die Korbstruktur (10) zurückgebogen sind.

16. Implantat nach einem der vorstehenden Ansprüche, wobei Haltering (4), Korbstruktur (10) und Ankerelemente (7b) aus einem Formgedächtnismetall bestehen.

## Claims

1. Medical implant (1) for the occlusion of a patient's auricula sinistra by endovascular means, said implant having a cage structure (10), the cage structure comprising a plurality of webs (3) that are proximally attached to a retaining ring (4) via connecting webs (2) the cage structure being open distally and limited by a rim of converging webs (3), wherein said implant (1) consists of a self-expanding material, has the shape of a slotted tube in a contracted state and, after expansion, assumes the cage structure (10) of a diameter larger than that of the retaining ring (4), wherein upon expansion several anchor elements (7) are arranged within or distally to the cage structure (10), said anchor elements being proximally connected directly with the retaining ring (4), wherein the anchor elements (7b) project laterally beyond the cage structure (10) and extend in proximal direction in a curved configuration with the intention to be laterally supported against the muscle tissue of the auricula sinistra.

2. Implant according to claim 1, wherein the anchor elements (7b) are equally spaced over the circumference of the retaining ring (4).

3. Implant according to any one of the preceding claims, wherein the anchor elements (7b) are arranged inside the cage structure and are directly attached to the retaining ring (4).

4. Implant according to any one of the preceding claims, wherein the connecting webs (2) extending from retaining ring (4) towards the cage structure (10) are provided with perforations (6).

5. Implant according to any one of the preceding claims, wherein the implant further comprises a cover arranged in a proximal region.

6. Implant according to claim 5, wherein the cover consists of a Teflon film or coat.

7. Implant according to claim 5 or 6, wherein the cover is attached to the cage structure (10) by sewing making use of the perforations.

8. Implant according to any one of the preceding claims, wherein the cage structure (10) is composed of a meshwork of branching and converging webs (3).

9. Implant according to any one of the preceding claims, wherein the distal rim of the cage structure (10) has rounded tips (5).

10. Implant according to any one of the preceding claims, wherein the implant further comprises a coupling for a guide catheter on the retaining ring (4).

11. Implant according to any one of the preceding claims, wherein the cage structure (10) is provided in a proximal area with a central deepening portion in which the retaining ring (4) is arranged.

12. Implant according to any one of the preceding claims wherein each of the anchor elements (7b) is arranged between two connecting webs (2) at the retaining ring (4).

13. Implant according to any one of the preceding claims, wherein the anchor elements (7b) are provided with rounded ends (12).

14. Implant according to claim 1, wherein the anchor elements (7b) are arranged distally to the cage structure (10), project laterally beyond the cage structure (10), and are connected to the retaining ring (4) via a retaining tube (14).

15. Implant according to claim 14, wherein neighboring anchor elements (7b) are connected with each other in pairs,
wherein the tips of the anchor elements (7b) are bent inwardly pointing towards the cage structure (10).

16. Implant according to any one of the preceding claims, wherein retaining ring (4), cage structure (10), and anchor elements (7b) consist of a shape-memory metal.

## Revendications

1. Implant médical (1) destiné à verrouiller l'auricule gauche d'un patient par voie endovasculaire, qui présente une structure de panier (10) composée d'une pluralité d'entretoises (3), qui est reliée à une bague de retenue (4) côté proximal par l'intermédiaire d'entretoises de liaison (2) et qui est ouverte côté distal et est délimitée par une couronne d'entretoises (3) convergentes, dans lequel l'implant (1) est constitué d'un matériau auto-expansible, présente dans un état contracté la forme d'un tube fendu et adopte, après l'expansion, la structure de panier (10) avec un diamètre agrandi par rapport à la bague de retenue (4), dans lequel sont disposés après l'expansion à l'intérieur de la structure de panier (10) ou côté distal par rapport à la structure de panier (10) plusieurs éléments d'ancrage (7), qui sont reliés côté proximal directement à la bague de retenue (4) et les éléments d'ancrage (7b) font saillie de manière latérale au-delà de la structure de panier (10), s'étendent de manière recourbée vers le côté proximal et se destinent à prendre appui de manière latérale contre le tissu musculaire de l'auricule gauche.

2. Implant selon la revendication 1, dans lequel les éléments d'ancrage (7b) sont disposés de manière homogène sur la périphérie de la bague de retenue (4).

3. Implant selon l'une quelconque des revendications précédentes, dans lequel les éléments d'ancrage (7b) sont disposés à l'intérieur de la structure de panier et sont reliés directement à la bague de retenue (4).

4. Implant selon l'une quelconque des revendications précédentes, dans lequel les entretoises de liaison (2) partant de la bague de retenue (4) présentent vers la structure de panier (10) des perforations (6).

5. Implant selon l'une quelconque des revendications précédentes, dans lequel l'implant présente par ailleurs un recouvrement dans la zone proximale.

6. Implant selon la revendication 5, dans lequel le recouvrement est constitué d'un film en téflon.

7. Implant selon la revendication 5 ou 6, dans lequel le recouvrement est cousu à la structure de panier (10) par l'intermédiaire des perforations.

8. Implant selon l'une quelconque des revendications précédentes, dans lequel la structure de panier (10) est formée par un réseau d'entretoises (3) de ramification convergentes.

9. Implant selon l'une quelconque des revendications précédentes, dans lequel la couronne distale de la structure de panier (10) présente des pointes arrondies (5).

10. Implant selon l'une quelconque des revendications précédentes, dans lequel l'implant présente par ailleurs
un couplage pour un cathéter de guidage sur la bague de retenue (4).

11. Implant selon l'une quelconque des revendications précédentes, dans lequel la structure de panier (10) présente dans la zone proximale un creux central, dans lequel la bague de retenue (4) est disposée.

12. Implant selon l'une quelconque des revendications précédentes, dans lequel les éléments d'ancrage (7b) sont disposés sur la bague de retenue (4) respectivement entre deux entretoises de liaison (2).

13. Implant selon l'une quelconque des revendications précédentes, dans lequel les éléments d'ancrage (7b) présentent des extrémités arrondies (12).

14. Implant selon la revendication 1, dans lequel les éléments d'ancrage (7b) sont disposés côté distal par rapport à la structure de panier (10), font saillie de manière latérale au-delà de la structure de panier (10) et sont reliés à la bague de retenue (4) par l'intermédiaire d'un tube de retenue (14).

15. Implant selon la revendication 14, dans lequel
des éléments d'ancrage (7b) adjacents sont reliés les uns aux autres par paires, dans lequel les pointes des éléments d'ancrage (7b) sont repliées en direction de la structure de panier (10).

16. Implant selon l'une quelconque des revendications précédentes, dans lequel la bague de retenue (4), la structure de panier (10) et les éléments d'ancrage (7b) sont constitués d'un métal à mémoire de forme.
